# DEMANDE DE BREVET EUROPEEN

(11) **EP 0 693 290 A1**
(43) Date de publication de la demande: **24.01.1996**
(21) Numéro de dépôt: 95401716.6
(22) Date de dépôt: 19.07.1995
(51) Int. Cl.: A61L 15/22, A61L 15/42

(54) **Pansement hydrocolloide hémostatique**

(30) Priorité: 20.07.1994 FR 9408990
(71) Demandeur: LABORATOIRES D'HYGIENE ET DE DIETETIQUE, F-75008 Paris (FR)
(72) Inventeur: Janod, Philippe, F-39100 Champvans (FR)
(74) Mandataire: Clisci, Serge

(57) **Abrégé**

La présente invention concerne l'utilisation de la matrice d'un pansement hydrocolloïde, ladite matrice étant essentiellement constituée d'un réseau élastomérique adhésif dans lequel est inséré au moins un composé hydrocolloïde, en tant qu'agent hémostatique topique.

## Description

### Domaine de l'invention

La présente invention concerne l'utilisation d'un pansement hydrocolloïde en tant qu'agent hémostatique topique pour les plaies hémorragiques.

### Description de l'art antérieur

L'expression pansement hydrocolloïde est un terme générique qui désigne un pansement à usage topique, ayant généralement la forme d'une plaque ou stratifié, comprenant un support et une matrice constituée d'une phase hydrophile colloïdale et d'une phase hydrophobe adhésive. Par "masse hydrocolloïde" ou "matrice hydrocolloïde", on entend ici la matrice dudit pansement hydrocolloïde.

On trouvera par exemple des descriptions de pansements et matrice hydrocolloïdes dans le brevet français FR-B-2 495 473 et le brevet américain US-A-3 339 546, inclus ici à titre de références.

Les pansements hydrocolloïdes sont utilisés dans les soins des plaies secondaires à un traumatisme ou à une affection entraînant une perte de substance. Ils sont notamment utilisés dans le traitement des plaies profondes non-hémorragiques comme les plaies chroniques telles que les ulcères veineux ou artériels, les escarres et les plaies aiguës comme les brûlures du premier degré et du deuxième degré superficiel, les dermabrasions cutanées thérapeutique ou esthétique ainsi que les plaies chirurgicales ou superficielles.

L'intérêt des pansements hydrocolloïdes est leur aptitude à favoriser le processus de cicatrisation. En effet, grâce à leur forte capacité d'absorption des exsudats, les pansements hydrocolloïdes forment au contact de la plaie un gel humide et non adhérent favorable au processus cicatriciel sans que le tissu néo-formé soit endommagé au cours du renouvellement du pansement. Par ailleurs, de par leurs propriétés adhésives les pansements-hydrocolloïdes adhèrent aux pourtours de la plaie et peuvent rester en place pendant plusieurs jours sans inconfort pour le patient.

Pour traiter les plaies hémorragiques, l'homme du métier a naturellement pensé (i) à incorporer un agent hémostatique dans la matrice hydrocolloïde du pansement hydrocolloïde, ou (ii), solution retenue par les médecins, à répartir sur lesdites plaies hémorragiques un agent hémostatique et revêtir lesdites plaies ainsi traitées avec un pansement hydrocolloïde.

Les pansements hydrocolloïdes sont principalement utilisés à l'heure actuelle en tant que joints pour les poches d'ostomies.

On sait par ailleurs que certaines substances hydrocolloïdes, telles que le chitosan, présentent des propriétés spécifiques hémostatiques. Voir à cet effet la demande européenne publiée EP-A-0 340 943 et l'article de W.G. MALETTE et al. intitulé "Chitosan : A New Hemostatic" et publié dans The Annals of Thoracic Surgery 36 (No 1), pages 55-58, (1983).

### Objet de l'invention

On vient à présent de découvrir, de façon surprenante, que les pansements hydrocolloïdes, bien connus de l'art antérieur en tant que pansements aptes à favoriser le processus de cicatrisation de plaies non-hémorragiques, possèdent également la propriété d'être hémostatiques, sur des plaies hémorragiques, sans qu'il soit nécessaire d'incorporer dans la matrice hydrocolloïde un agent hémostatique spécifique.

On a observé, selon l'invention, que les matrices hydrocolloïdes constituées essentiellement de deux composants : un composant élastomère adhésif, et un composant hydrocolloïde réputé comme étant non-hémostatique *per se*, donnent des pansements hémostatiques particulièrement efficaces sur des plaies hémorragiques.

On a en particulier démontré, selon les expérimentations décrites ci-après, que les pansements hydrocolloïdes diminuent le temps de coagulation du sang et activent l'adhésion plaquettaire.

Selon la présente invention, on préconise une nouvelle utilisation de la matrice d'un pansement hydrocolloïde, ladite matrice étant essentiellement constituée d'un réseau élastomérique adhésif dans lequel est inséré au moins un composé hydrocolloïde, choisi parmi l'ensemble constitué par l'alcool polyvinylique, la pectine, la gélatine, la carboxyméthylcellulose, la carboxyméthylcellulose sodique et leurs mélanges, en tant qu'agent hémostatique topique pour les plaies hémorragiques.

Cette matrice est liée à une couche support.

En d'autres termes, on vise selon l'invention, l'utilisation d'un composant hydrocolloïde non spécifiquement hémostatique, pour la préparation d'une matrice hémostatique constituée essentiellement de deux composants, ledit composant hydrocolloïde et un composant élastomère adhésif, ladite matrice étant destinée à être utilisée en tant que pansement vis-à-vis des plaies hémorragiques.

### Description détaillée de l'invention

Les matrices qui conviennent selon l'invention sont celles qui sont connues de l'art antérieur dans le domaine des pansements hydrocolloïdes et qui comprennent, liée à un support, une matrice hydrocolloïde essentiellement constituée d'un réseau élastomérique adhésif dans lequel est inséré au moins un composé hydrocolloïde.

Le support constitue la face externe du pansement. Il est en général constitué d'un non-tissé, d'une mousse ou d'un film de polymère synthétique.

Le polymère préféré pour le support est le polyuréthanne qui possède des propriétés mécaniques en partie responsables des performances du pansement. De par sa plasticité et sa résistance à l'étirement, le polyuréthanne permet d'obtenir un support tel que le pansement se conforme à l'anatomie de la plaie à protéger.

L'imperméabilité aux liquides et aux bactéries et, à l'opposé, la perméabilité aux gaz et notamment à l'oxygène, font également du polyuréthanne un matériau de choix en tant que support des pansements hydrocolloïdes.

La matrice est constituée d'un réseau élastomérique adhésif dans lequel sont insérés un ou plusieurs hydrocolloïdes.

Le réseau élastomérique constitue la phase hydrophobe de la matrice et lui confère son adhésivité. Les polymères préférés constituant cette phase sont les copolymères blocs styrène-oléfine-styrène, notamment les copolymères styrène-isoprène-styrène, les copolymères styrène-butylène-styrène et les copolymères styrène-éthylène-butylène-styrène, les copolymères blocs éthylène-propylène, d'une part, le polyisobutylène, les copolymères d'éthylène et d'acétate de vinyle, d'autre part, et leurs mélanges.

Les hydrocolloïdes insérés dans le réseau élastomérique sont des polymères naturels ou synthétiques, notamment l'alcool polyvinylique, la pectine, la gélatine, les carboxyalkylcelluloses, notamment la carboxyméthylcellulose, la carboxyméthylcellulose sodique et leurs mélanges.

Ces hydrocolloïdes constituent la phase hydrophile de la matrice qui peut ainsi absorber jusqu'à onze fois son poids d'eau en 24 heures en se transformant en un gel à viscosité élevée.

Les pansements hydrocolloïdes sont présentés sous forme de plaques de un à quelques millimètres d'épaisseur et de surface comprise entre 50 cm et 500 cm. Leur densité varie en général d'environ 0,7 à environ 1 g/cm³.

A titre d'exemple, on peut citer les pansements hydrocolloïdes suivants qui conviennent selon l'utilisation de l'invention :
COMFEEL® commercialisé par les Laboratoires COLOPLAST, ALGOPLAQUE® commercialisé par les Laboratoires URGO, DUODERM® commercialisé par les Laboratoires CONVATEC et BIOFILM® commercialisé par les Laboratoires BIOTROL PHARMA.

### Meilleur mode

Le meilleur mode de mise en oeuvre de l'invention consiste à faire appel à un pansement hydrocolloïde comprenant un support constitué d'un film de polyuréthanne et d'une matrice hydrocolloïde constitué de (i) polyisobutylène et de copolymère styrène-isoprène-styrène, et (ii) de carboxyméthylcellulose sodique, de pectine et de gélatine.

### Expérimentations

On a évalué les propriétés hémostatiques des pansements hydrocolloïdes.

Pour ce faire, on a mesuré le temps global de coagulation de sang humain complet non anticoagulé provenant de trois donneurs volontaires. Le prélèvement de sang est réalisé au niveau de la veine du pli du coude par ponction franche au moyen d'une aiguille de 1,1 mm de diamètre. Le sang de chaque donneur est réparti sous quatre volumes de 7 ml dans des tubes de polystyrène dont la paroi interne est recouverte avec un des échantillons à tester de pansements hydrocolloïdes de 6,5 x 4,2 cm. La surface des pansements destinée à être en contact avec la peau est tournée vers la lumière du tube. Trois tubes sont recouverts avec ALGOPLAQUE®, trois tubes sont recouverts avec DUODERM®, trois tubes sont recouverts avec COMFEEL® et trois tubes témoins ne sont pas recouverts (tubes secs). Tous les tubes sont placés au bain marie à 37°C et bouchés avec du PARAFILM®. Toutes les minutes, la présence d'un caillot est recherchée par observation visuelle avec léger retournement du tube. Le temps de coagulation est noté pour chacun des tubes. On a regroupé dans le tableau I les résultats obtenus. Dans ce tableau, les temps de coagulation sont exprimés en minutes.

Il ressort de ces résultats que la présence des pansements hydrocolloïdes accélère le processus de coagulation.

Les temps de coagulation sont diminués d'environ 50% en présence de ALGOPLAQUE® et DUODERM® et de façon légèrement moins prononcée en présence de COMFEEL®.

Les propriétés hémostatiques des pansements hydrocolloïdes seront avantageusement mises à profit pour la fabrication de pansements individuels destinés à traiter les plaies particulièrement exsudatives et hémorragiques telles que les dermabrasions, les sites donneurs de greffe cutanée, les plaies chirurgicales hémorragiques ainsi que toutes plaies hémorragiques (ampoules, coupures...).

**TABLEAU I**

| **Temps de coagulation (en minute)** | | | |
|---|---|---|---|
| | **Donneur 1** | **Donneur 2** | **Donneur 3** |
| **Témoin (tube sec)** | 17 | 19 | 15 |
| **ALGOPLAQUE** ® **HP** | 7 | 8 | 8 |
| **DUODERM** ® | 7 | 11 | 7 |
| **COMFEEL** ® | 9 | 10 | 13 |

### EXEMPLES 1-5

A titre d'illustration et de façon nullement limitative, des pansements ont été préparés par dépôt (300 à 1400 g/m en poids sec) d'une matrice constituée de 50 à 63 % en poids de composant élastomère adhésif, de 50 à 36 % en poids de composant hydrocolloïde et le cas échéant de 1 % en poids d'agent antioxydant. Les formulations sont les suivantes avec les abréviations :
- CMC Na: : carboxyméthylcellulose sodique
- EVA: : copolymère éthylène-acétate de vinyle
- PIB: : polyisobutylène
- SBS: : copolymère tribloc styrène-butadiène-styrène
- SIS: : copolymère tribloc styrène-isoprène-styrène

### Exemple 1

| Composant élastomère | |
|---|---|
| PIB (produit commercialisé sous la nomenclature de "LMMN" par la société dite EXXON) | 28 % en poids |
| PIB (produit commercialisé sous la nomenclature de "LMMH" par la société dite EXXON) | 24 % en poids |

| Composant hydrocolloïde | |
|---|---|
| CMC Na | 30 % en poids |
| Pectine | 12 % en poids |
| Gélatine | 6 % en poids |

### Exemple 2

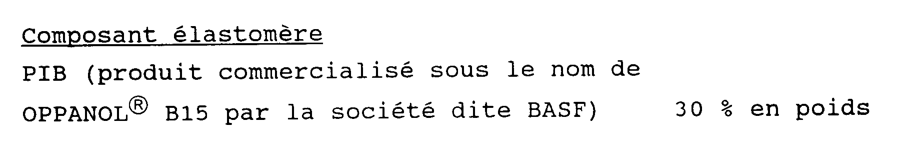

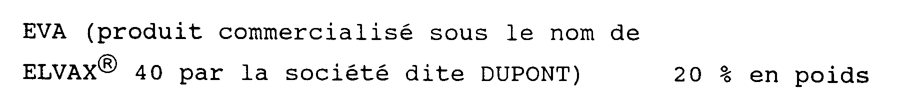

| Composant hydrocolloïde | |
|---|---|
| CMC Na | 30 % en poids |
| Pectine | 8 % en poids |
| Gélatine | 12 % en poids |

### Exemple 3

| Composant élastomère | |
|---|---|
| PIB (produit "LMMN" comme indiqué ci-dessus) | 20 % en poids |
| PIB (produit "LMMH" comme indiqué ci-dessus) | 20 % en poids |
| SIS (produit commercialisé sous le nom de CARIFLEX® TR 1107 par la société dite SHELL) | 12 % en poids |

| Composant hydrocolloïde | |
|---|---|
| CMC Na | 30 % en poids |
| Pectine | 10 % en poids |
| Gélatine | 8 % en poids |

### Exemple 4

| Composant élastomère | |
|---|---|
| SIS (produit CARIFLEX® TR 1107 comme indiqué ci-dessus) | 14 % en poids |
| FORAL® 85 (résine ester de colophane avec le glycérol commercialisé par la société dite HERCULES) | 34 % en poids |
| Paraffine | 15 % en poids |

| Composant hydrocolloïde | |
|---|---|
| CMC Na | 26 % en poids |
| Pectine | 10 % en poids |

| Composant antioxydant | |
|---|---|
| IRGANOX® 1010 (commercialisé par la société dite CIBA | 1 % en poids |

### Exemple 5

| | |
|---|---|
| produit commercialisé sous SIS (produit CARIFLEX® TR 1107 comme indiqué ci-dessus) | 15 % en poids |
| SBS (produit commercialisé sous le nom de CARIFLEX® TR 1102 par la société dite SHELL) | 5 % en poids |
| STAYBELITE® ESTER 10 (résine de dérivé de colophane commercialisée par la société dite HERCULES) | 20 % en poids |
| ESCOREZ® 1310 (résine tackifiante commercialisée par la société dite EXXON) | 5 % en poids |
| Paraffine | 15 % en poids |

| Composant hydrocolloïde | |
|---|---|
| Pectine | 14 % en poids |
| Gélatine | 5 % en poids |
| CMC Na | 20 % en poids |

| Composant antioxydant | |
|---|---|
| IRGANOX® 1010 (commercialisé par la société dite CIBA | 1 % en poids |

Les pansements obtenus ont été consignés dans le tableau II ci-après. Ces pansements hydrocolloïdes se sont révélés être particulièrement efficace sur les plaies hémorragiques eu égard à leurs propriétés hémostatiques.

## Revendications

1. Utilisation de la matrice d'un pansement hydrocolloïde, ladite matrice étant essentiellement constituée d'un réseau élastomérique adhésif dans lequel est inséré au moins un composé hydrocolloïde, choisi parmi l'ensemble constitué par l'alcool polyvinylique, la pectine, la gélatine, la carboxyméthylcellulose, la carboxyméthylcellulose sodique et leurs mélanges, en tant qu'agent hémostatique topique.

2. Utilisation selon la revendication 1, caractérisée en ce que le réseau élastomérique adhésif est constitué d'un polymère synthétique choisi parmi l'ensemble constitué par les copolymères blocs styrène-oléfine-styrène, les copolymères blocs éthylène-propylène, le polyisobutylène, les copolymères d'éthylène et d'acétate de vinyle et leurs mélanges.

3. Utilisation selon la revendication 1, caractérisée en ce que le composé hydrocolloïde est choisi parmi l'ensemble constitué par la pectine, la gélatine, la carboxyméthylcellulose sodique et leurs mélanges.

4. Utilisation selon la revendication 1, caractérisée en ce que ladite matrice est liée à un support constitué d'un film de polyuréthanne.

5. Utilisation selon la revendication 1, caractérisée en ce que l'on fait appel à un pansement hydrocolloïde comprenant un support constitué d'un film de polyuréthanne et d'une matrice hydrocolloïde constitué de (i) polyisobutylène et de copolymère styrène-isoprène-styrène, et (ii) de carboxyméthylcellulose sodique, de pectine et de gélatine.

6. Utilisation d'un composant hydrocolloïde non spécifiquement hémostatique, pour la préparation d'une matrice hémostatique constituée essentiellement de deux composants, ledit composant hydrocolloïde et un composant élastomère adhésif, ladite matrice étant destinée à être utilisée en tant que pansement vis-à-vis des plaies hémorragiques.
